(19) Europäisches Patentamt
European Patent Office
Office européen des brevets

(11) Publication number: 0 246 061 B1

(12) # EUROPEAN PATENT SPECIFICATION

(45) Date of publication of patent specification: **18.03.92**

(51) Int. Cl.5: **C07C 275/54**, C07C 211/52, C07C 265/12, A01N 47/34

(21) Application number: **87304196.6**

(22) Date of filing: **12.05.87**

(54) **A benzoylurea derivative and its production and use.**

(30) Priority: **13.05.86 JP 110232/86**
**31.07.86 JP 181250/86**

(43) Date of publication of application:
**19.11.87 Bulletin 87/47**

(45) Publication of the grant of the patent:
**18.03.92 Bulletin 92/12**

(84) Designated Contracting States:
**BE CH DE ES FR GB IT LI NL**

(56) References cited:
**EP-A- 0 008 435**

(73) Proprietor: **SUMITOMO CHEMICAL COMPANY, LIMITED**
**Kitahama 4-chome 5-33**
**Chuo-ku Osaka 541(JP)**

(72) Inventor: **Tanabe, Yoo**
**10-3-342 Sonehigashinocho-2-chome**
**Toyonaka-shi(JP)**
Inventor: **Ohsumi, Tadashi**
**7-10-202, Kawazoecho**
**Nishinomiya-shi(JP)**
Inventor: **Yano, Toshihiko**
**15-10-103, Kusunokicho**
**Ashiya-shi(JP)**
Inventor: **Takada, Yoji**
**14-7, Mefu-2-chome**
**Takarazuka-shi(JP)**

(74) Representative: **Harrison, David Christopher et al**
**MEWBURN ELLIS & CO 2/3 Cursitor Street**
**London EC4A 1BO(GB)**

EP 0 246 061 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a novel benzoylurea derivative represented by the formula (I),

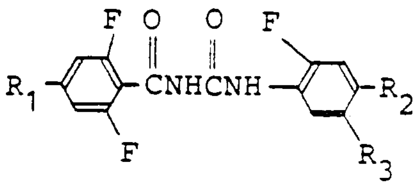

(I)

wherein $R_1$ is a hydrogen atom or a fluorine atom, $R_2$ is a trifluoromethyl group, a pentafluoroethyl group or a heptafluoropropyl group, and $R_3$ is a hydrogen atom, a fluorine atom or a chlorine atom, its production and insecticides containing it as an active ingredient.

We have made many studies directed towards the development of excellent insecticides, and as a result, found that the benzoylurea derivatives represented by the formula (I) (hereinafter referred to as "present compound") have excellent properties in that they have a high insecticidal activity against the larvae of insect pests and can be produced at a relatively low cost.

Preferred present compounds represented by the formula (I) are those in which $R_1$ is a hydrogen atom or a fluorine atom, $R_2$ is a trifluoromethyl group, a pentafluoroethyl group or a heptafluoropropyl group and $R_3$ is a hydrogen atom or a fluorine atom.

Known benzoylurea compounds having an insecticidal activity are, for example, diflubenzuron, penfluron both are described in JP-A-18255/1977), teflubenzuron [JP-A-126460/1982] and compounds described in JP-A-106454/1984 and JP-A-76452/1986. However, it was recently found that the present compounds have a higher insecticidal activity than that of the well-known compounds.

As insect pests against which the present compounds are particularly effective, the following specific examples may be given: Larvae of insect pests belonging to Lepidoptera such as diamond-back moth (Plutella xylostella), rice stem borer (Chilo suppressalis), armyworms and cutworms, Plusiid moth (Plusiinae), small white butterfly (Pieris rapae crucivora), casemaking clothes moth (Tinea pellionella), webbing clothes moth (Tineola bisselliella); larvae of insect pests belonging to Diptera such as common mosquito (Culex pipiens pallens), Anopheline mosquitoes, Aedes sp., chironomid midges, housefly (Musca domestica), blackflies; nymphs of insect pests belonging to Dictyoptera such as German cockroach (Blattella germanica), smoky-brown cockroach (Periplaneta fuliginosa), brown cockroach (Periplaneta brunnea), American cockroach (Periplaneta americana); and larvae of other insect pests belonging to Coleoptera or Hymenoptera.

Examples of the present compound are given below.

N-2,6-difluorobenzoyl-N'-2-fluoro-4-trifluoromethylphenylurea
N-2,6-difluorobenzoyl-N'-2,5-difluoro-4-trifluoromethylphenylurea
N-2,4,6-trifluorobenzoyl-N'-2-fluoro-4-trifluoromethylphenylurea
N-2,4,6-trifluorobenzoyl-N'-2,5-difluoro-4-trifluoromethylphenylurea
N-2,6-difluorobensoyl-N'-2-fluoro-4-pentaluoroethylnhenylurea
N-2,6-difluorobenzoyl-N'-2,5-difluoro-4-pentafluoroethylphenylurea
N-2,4,6-trifluorobenzoyl-N'-2-fluoro-4-pentafluoroethylphenylurea
N-2,6-difluorobenzoyl-N'-2,5-difluoro-4-heptafluoropropylphenylurea
N'-2,4,6-trifluorobenzoyl-N'-2,5-difluoro-4-heptafluoropropylphenylurea
N-2,6-difluorobenzoyl-N'-2-fluoro-4-heptafluoropropylphenylurea
N-2,4,6-trifluorobenzoyl-N'-2-fluoro-4-heptafluoropropylphenylurea
N-2,4,6-trifulorobenzoyl-N'-2,5-difluoro-4-pentafluoroethylphenylurea
N-2,6-difluorobenzoyl-N'-5-chloro-2-fluoro-4-trifluoromethylphenylurea
N-2,4,6-trifluorobenzoyl-N'-5-chloro-2-fluoro-4-trifluoromethylphenylurea
N-2,6-difluorobenzoyl-N'-5-chloro-2-fluoro-4-pentafluoroethylphenylurea
N-2,4,6-trifluorobenzoyl-N'-5-chloro-2-fluoro-4-pentafluoroethylphenylurea
N-2,6-difluorobenzoyl-N'-5-chloro-2-fluoro-4-heptafluoropropylphenylurea
N-2,4,6-trifluorobenzoyl-N'-5-chloro-2-fluoro-4-heptafluoropropylphenylurea

The present compounds can be produced, for example, by a method of reacting a benzoylisocyanate

compound represented by the formula (II),

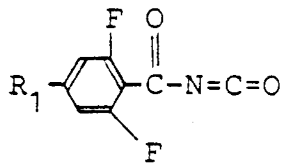

(II)

wherein $R_1$ is as defined above, with an aniline compound represented by the formula (III),

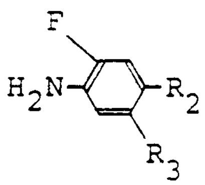

(III)

wherein $R_2$ and $R_3$ are as defined above, (hereinafter referred to as Method A), or by a method of reacting a benzamide compound represented by the formula (IV),

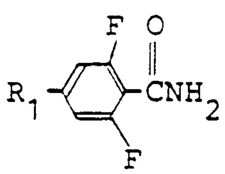

(IV)

wherein $R_1$ is as defined above, with an isocyanate compound represented by the formula (V),

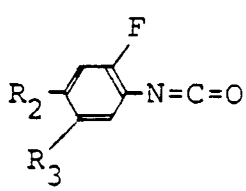

(V)

wherein $R_2$ and $R_3$ are as defined above, (hereinafter referred to as Method B).

In the above methods, an inert solvent is generally used in order to allow the reaction to proceed smoothly. Such solvents include, for example, hydrocarbons (e.g. benzene, toluene, xylene, nitromethane), halogenated hydrocarbons (e.g. chlorobenzene, carbon tetrachloride, chloroform, methylene chloride, 1,2-dichloroethane), ethers (e.g. diethyl ether, tetrahydrofuran, dioxane), ketones (e.g. acetone, methyl ethyl ketone, methyl isobutyl ketone), polar organic solvents (e.g. dimethyl sulfoxide, dimethylformamide, sulfolane) and mixtures of two or more of them. The amounts in which the reagents are generally used provide an equimolar ratio, but either one of them may be in excess of the other. The reaction temperature is not particularly limited, but may be in a range from 0° to 80°C, generally from room temperature to 60°C for Method A, and in a range from room temperature to 180°C, generally from 80° to 160°C for Method B. The reaction time depends upon the reaction temperature, amount of the reagent used and kind of the reaction solvent, but generally a range from 1 to 50 hours is sufficient to attain the object.

The present compounds obtained can be purified by means of column chromatography, recrystallization, etc. as need arises.

Among the aniline compounds represented by the formula (III), both dihalogenated trifluoromethylaniline represented by the formula (III′),

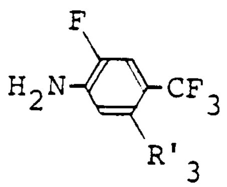

(III')

wherein $R'_3$ is a fluorine atom or a chlorine atom, and a fluorine-containing aniline compound represented by the formula (III″),

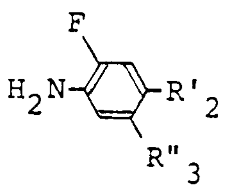

(III")

wherein $R'_2$ is a pentafluoroethyl group or a heptafluoropropyl group, and $R''_3$ is a hydrogen atom, a fluorine atom or a chlorine atom, are novel compounds.

The aniline compounds represented by the formula (III) can be produced, for example, by a novel method as described below, i.e. by reacting an acetanilide compound represented by the formula (VI),

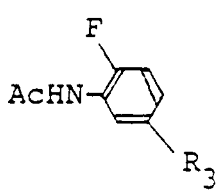

(VI)

wherein $R_3$ is as defined above, with a carboxylic acid represented by the formula (VII),

$R_2CO_2H$ (VII)

wherein $R_2$ is as defined above, in the presence of xenon difluoride, to afford the resulting acetanilide compound represented by the formula (VIII),

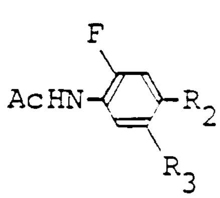

(VIII)

wherein $R_2$ and $R_3$ are as defined above, followed by deacetylation to give the desired compound represented by the formula (III).

The amounts of both xenon difluoride and the carboxylic acid (VII), i.e. trifluoroacetic acid, pentafluoropropionic acid or heptafluorobutyric acid, used in a reaction for introducing a trifluoromethyl, pentafluoroethyl or heptafluoropropyl group, are generally from 1 to 5 times based on the amount of the acetanilide compound (VI). In this reaction, dichloromethane or other halogenated hydrocarbons are generally used as solvent. As to the reaction temperature, a range from -10° to 50°C is sufficient to attain the object.

The reaction product thus obtained can be easily purified by column chromatography on silica gel, etc. as need arises.

Deacetylation is generally carried out under an acidic condition. For the acid, 5 to 35% aqueous hydrochloric acid solution, 5 to 80% aqueous sulfuric acid solution and other aqueous mineral acid solutions may be used, and as need arises, alcohol solvents (e.g. methanol, ethanol) or other organic solvents (e.g. tetrahydrofuran, acetonitrile) may also be used together with the acid solution. In this reaction, the reaction temperature may be in a range from room temperature to the boiling point of the solvent. The product obtained can be easily purified by distillation, etc. as need arises.

The isocyanate compound represented by the formula (V) can be easily produced by a conventional method, for example, by reacting an aniline compound represented by the formula (III) with phosgene.

In this reaction, the amount of phosgene used is generally from 1 to 5 times based on the aniline compound (III), and an inert solvent is generally used. Generally, such inert solvents include, for example, hydrocarbons (e.g. hexane, heptane, benzene, toluene), halogenated hydrocarbons (e.g. dichloromethane, chloroform, 1,2-dichloroethane, chlorobenzene) and mixtures of two or more of them. This reaction proceeds to a sufficient degree in a range from room temperature to the boiling point of the solvents. The reaction product thus obtained can be easily purified by distillation, etc. as need arises.

When the present compounds are used as an active ingredient for insecticides, they may be used as they are without adding any other ingredients. Generally, however, they are formulated into emulsifiable concentrates, wettable powders, dusts, granules, oil sprays, aerosols, poisonous baits, etc. by mixing with solid carriers, liquid carriers, gaseous carriers, surface active agents, other auxiliaries for formulation, baits, etc.

Thus, the invention additionally provides an insecticidal composition containing a present compound and a diluent or carrier. In such a preparation, the content of the present compound, which is an active ingredient, is preferably from 0.01 to 95% by weight. The solid carrier includes for example fine powders or granules of kaoline clay, attapulgite clay, bentonite, terra alba, pyrophyllite, talc, diatomaceous earth, calcite, corn stalk powder, walnut shell powder, urea, ammonium sulfate and synthetic hydrated silicon dioxide. The liquid carrier includes for example aliphatic hydrocarbons (e.g. kerosene), aromatic hydrocarbons (e.g. benzene, toluene, xylene, methylnaphthalene), halogenated hydrocarbons (e.g. dichloroethane, trichloroethylene, carbon tetrachloride), alcohols (e.g. ethylene glycol, cellosolve), ketones (e.g. acetone, methyl ethyl ketone, cyclohexanone, isophorone), ethers (e.g. diethyl ether, dioxane, tetrahydrofuran), esters (e.g. ethyl acetate), nitriles (e.g. acetonitrile, isobutyronitrile), acid amides (e.g. dimethylformamide, dimethylacetamide), dimethyl sulfoxide and vegetable oils (e.g. soybean oil, cotton seed oil). The gaseous carrier includes for example freon gas, LPG (liquefied petroleum gas) and dimethyl ether. The surface active agent used for emulsification, dispersion, wetting, etc. includes for example anionic surface active agents such as the salt of alkyl sulfates, alkyl(aryl)sulfonates, dialkyl sulfosuccinates, the salt of polyoxyethylene alkylaryl ether phosphoric acid ester, naphthalenesulfonic acid/formalin condensates, and nonionic surface active agents such as polyoxyethylene alkyl ether, polyoxyethylene polyoxypropylene block copolymers, sorbitan fatty acid esters, polyoxyethylene sorbitan fatty acid esters. The auxiliary for formulation such as fixing agents, dispersing agents, etc. includes for example lignosulfonates, alginates, polyvinyl alcohol, gum arabic, molasses, casein, gelatin, CMC (carboxymethyl cellulose), pine oil and agar. The stabilizer includes for example alkyl phosphates [e.g. PAP (isopropyl acid phosphate), TCP (tricresyl phosphate)], vegetable oils, epoxidized oils, the foregoing surface active agents, antioxidants (e.g. BHT, BHA), fatty acid salts (e.g. sodium oleate, calcium stearate) and fatty acid esters (e.g. methyl oleate, methyl stearate).

The preparations obtained may be used as they are or in dilution with water. They may also be used in a mixture with other insecticides, acaricides, nematocides, fungicides, herbicides, plant growth regulators, fertilizers, soil improvers, etc.

When the present compounds are used as insecticides, their dosage rate is generally from 0.5 to 500 g per 10 ares, and their application concentration is from 1 to 1000 ppm when emulsifiable concentrates, wettable powders, etc. are used in dilution with water. Dusts, granules, oil sprays, aerosols, etc. are used as

they are without dilution.

The present invention will be illustrated in more detail with reference to the following production examples, formulation examples and test examples.

Production examples of the present compounds will be shown.

Production example 1

[Production of the present compound (1)]

0.50 Gram of 2-fluoro-4-trifluoromethylaniline was dissolved in 10 ml of toluene, and to this solution, a solution of 0.51 g of 2,6-difluorobenzoylisocyanate in 5 ml of toluene was added dropwise with stirring and ice-cooling. After completion of the addition, stirring was continued overnight at room temperature and then 10 ml of hexane was added. The precipitated crystals were collected by filtration and dried to obtain 0.83 g of N-2,6-difluorobenzoyl-N′-2-fluoro-4-trifluoromethylphenylurea as white crystals.
m.p. 186.1 ° C
Yield 83%

Production example 2

[Production of the present compound (2)]

0.16 Gram of 2,6-difluorobenzamide and 0.22 g of 2,5-difluoro-4-trifluoromethylphenylisocyanate were added to 10 ml of xylene, and this mixture was refluxed for 20 hours. Thereafter, the reaction mixture was cooled, and the precipitated crystals were collected by filtration and recrystallized from acetone to give 0.23 g of N-2,6-difluorobenzoyl-N′-2,5-difluoro-4-trifluoromethylphenylurea as white crystals.
m.p. 204.0 ° C
Yield 61%

Production example 3

[Production of the present compound (3)]

0.20 Gram of 2,5-difluoro-4-trifluoromethylaniline was dissolved in 4 ml of toluene, and to this solution, a solution of 0.20 g of 2,4,6-trifluorobenzoylisocyanate in 2 ml of toluene was added dropwise with stirring and ice-cooling. After completion of the addition, stirring was continued overnight at room temperature and then 5 ml of hexane was added. The precipitated crystals were collected by filtration and dried to give 0.31 g of N-2,4,6-trifluorobenzoyl-N′-2,5-difluoro-4-trifluoromethylphenylurea as white crystals.
m.p. 171.6 ° C
Yield 78%

Production example 4

[Production of the present compound (6)]

150 Milligrams of 2,5-difluoro-4-pentafluoroethylaniline was dissolved in 5 ml of toluene, and to this solution, a solution of 110 mg of 2,6-difluorobenzoylisocyanate in 5 ml of toluene was added dropwise with stirring and ice-cooling. After completion of the addition, stirring was continued overnight at room temperature and then 10 ml of hexane was added. The precipitated crystals were collected by filtration and dried to give 153 mg of N-2,6-difluorobenzoyl-N′-2,5-difluoro-4-pentafluoroethylphenylurea as white crystals.
m.p. 162.7 ° C
Yield 59%

Production example 5

[Production of the present compound (7)]

49 Milligrams of 2,6-difluorobenzamide and 100 mg of 2,5-difluoro-4-heptafluoropropylphenylisocyanate were added to 2 ml of xylene, and this mixture was refluxed for 20 hours. Thereafter, the reaction mixture

was cooled, and the precipitated crystals were collected by filtration and recrystallized from acetone to give 87 mg of N-2,6-difluorobenzoyl-N′-2,5-difluoro-4-heptafluoropropylphenylurea as white crystals.
m.p. 183.5 ° C
Yield 58%

Production example 6

[Production of the present compound (8)]

150 Milligrams of 2,5-difluoro-4-heptafluoropropylaniline was dissolved in 5 ml of toluene, and to this solution, a solution of 102 mg of 2,4,6-trifluorobenzoylisocyanate in 2 ml of toluene was added dropwise with stirring and ice-cooling. After completion of the addition, stirring was continued overnight at room temperature and then 5 ml of hexane was added. The precipitated crystals were collected by filtration and dried to give 165 mg of N-2,4,6-trifluorobenzoyl-N′-2,5-difluoro-4-heptafluoropropylphenylurea as white crystals.
m.p. 194.4 ° C
Yield 65%

Production example 7

[Production of the present compound (9)]

95 Milligrams of 2-fluoro-4-pentafluoroethylaniline was dissolved in 5 ml of toluene, and to this solution, a solution of 83 mg of 2,6-difluorobenzoylisocyanate in 2 ml of toluene was added dropwise with stirring and ice-cooling. After completion of the addition, stirring was continued overnight at room temperature and then 10 ml of hexane was added. The precipitated crystals were collected by filtration and dried to obtain 132 mg of N-2,6-difluorobenzoyl-N′-2-fluoro-4-pentafluoroethylphenylurea as white crystals.
m.p. 154.6 ° C
Yield 74%

Some of the present compounds thus obtained are collectively shown in Table 1.

Table 1

Benzoylurea derivatives represented by the formula:

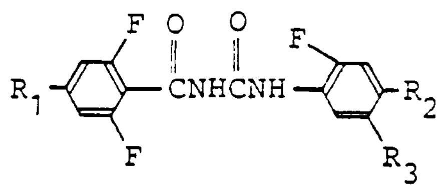

| Compound No. | Structure * | | | Physical constant |
|---|---|---|---|---|
| | $R_1$ | $R_2$ | $R_3$ | |
| (1) | H | $CF_3$ | H | mp.186.1°C |
| (2) | H | $CF_3$ | F | mp.204.0°C |
| (3) | F | $CF_3$ | F | mp.171.6°C |
| (4) | H | $CF_3$ | Cl | mp.187.3°C |
| (5) | F | $CF_3$ | H | mp.188.8°C |
| (6) | H | $C_2F_5$ | F | mp.162.7°C |
| (7) | H | $C_3F_7$ | F | mp.183.5°C |
| (8) | F | $C_3F_7$ | F | mp.194.4°C |
| (9) | H | $C_2F_5$ | H | mp.154.6°C |
| (10) | F | $C_2F_5$ | H | mp.118.2°C |

* Shows specific examples of the substituents, $R_1$, $R_2$ and $R_3$ of the benzoylurea derivatives represented by the above formula.

Production examples of the intermediates are described below

Production example 1

3.50 Grams of 2,5-difluoroacetanilide was dissolved in 40 ml of dichloromethane, and to this solution, 5.10 g of trifluoroacetic acid was added dropwise at room temperature with stirring. Thereafter, 7.5 g of xenon difluoride was added in small portions with water-cooling. After addition, stirring was continued overnight at room temperature, and after adding water, extraction was carried out with 100 ml of dichloromethane. The dichloromethane layer was washed with water and dried over anhydrous sodium

sulfate, and dichloromethane was removed by evaporation under reduced pressure to give 5.15 g of a product. This product was purified with column-chromatography on silica gel (solvent, chloroform : ethyl acetate = 20 : 1) to give 1.35 g of 2,5-difluoro-4-trifluoromethylacetanilide as white crystals.
Yield 25%
$^1$H-NMR (solvent, deutero chloroform; internal standard, TMS)
δ(ppm) 2.23 (s, 3H),
7.18 (dd, 1H, J = 6.0 Hz, 10.0 Hz),
7.70-8.20 (br., 1H),
8.21 (dd, 1H, J = 5.8 Hz, 12.0 Hz)
$^{19}$F-NMR (solvent, deutero chloroform; external standard, $CF_3CO_2H$)
δ(ppm) 18.0 (s, 3F),
37.0 (s, 1F),
54.0 (s, 1F).

Production example 2

1.35 Grams of 2,5-difluoro-4-trifluoromethylacetanilide was added to a mixed solvent of 10 ml of 20% sulfuric acid and 10 ml of methanol, followed by stirring under reflux at 80 ° C for 2 hours. The reaction mixture was then cooled and extracted with 50 ml of dichloromethane, and the dichloromethane layer was washed with water and dried over anhydrous sodium sulfate. After removing dichloromethane by evaporation under reduced pressure, the residue obtained was distilled to obtain 1.10 g of 2,5-difluoro-4-trifluoromethylaniline.
Yield 99%
b.p. 170- 172 ° C/15 mmHg
$^1$H-NMR (solvent, deutero chloroform; internal standard, TMS)
δ(ppm) 3.80-4.70 (br., 2H),
6.52 (dd, 1H, J = 8.0 Hz, 11.0 Hz),
7.16 (dd, 1H, J = 7.5 Hz, 12.0 Hz)

Production example 3

1.00 Gram of 5-chloro-2-fluoroacetanilide was dissolved in 20 ml of dichloromethane, and to this solution, 1.22 g of trifluoroacetic acid was added dropwise at room temperature with stirring. Thereafter, 1.80 g of xenon difluoride was added in small portions with water-cooling. After addition, stirring was continued overnight at room temperature, and after adding water, the reaction mixture was extracted with 50 ml of dichloromethane. The dichloromethane layer was washed with water and dried over anhydrous sodium sulfate, and dichloromethane was removed by evaporation under reduced pressure to give 1.38 g of a product. This product was column-chromatographed on silica gel (solvent, chloroform: ethyl acetate = 10 : 1) to obtain 0.27 g of 5-chloro-2-fluoro-4-trifluoromethylacetanilide as white crystals.
Yield 20%
$^1$H-NMR (solvent, deutero chloroform; internal standard, TMS)
δ(ppm) 2.25 (s, 3H),
7.42 (d, 1H, J = 11.0 Hz),
8.65 (d, 1H, J = 6.5 Hz).
$^{19}$F-NMR (solvent, deutero chloroform; external standard, $CF_3CO_2H$)
δ(ppm) -15.0 (s, 3F),
50.0 (s, 1F).

Production example 4

0.27 Gram of 5-chloro-2-fluoro-4-trifluoromethylacetanilide was added to a mixed solvent of 2 ml of 20% sulfuric acid and 2 ml of methanol, followed by stirring under reflux at 80 ° C for 2 hours. Thereafter, the reaction mixture was cooled and extracted with 5 ml of dichloromethane, and the dichloromethane layer was washed with water and dried over anhydrous sodium sulfate. After removing dichloromethane by evaporation under reduced pressure, the residue obtained was distilled to give 0.18 g of 5-chloro-2-fluoro-4-trifluoromethylaniline.
b.p. 180-185 ° C/13 mmHg

Yield 75%

Production example 5

Using the same procedure as that described in Production example 1, 2.00 g of 2,5-difluoroacetanilide, 4.18 g of pentafluoropropionic acid and 4.30 g of xenon difluoride gave 0.57 g of 2,5-difluoro-4-pentafluoroethylacetanilide as white crystals.
Yield 16%
$^1$H-NMR (solvent, deutero chloroform; internal standard, TMS)
δ(ppm) 2.30 (s, 3H),
7.32 (dd, 1H, J = 6.5 Hz, 10.0 Hz),
8.00-8.30 (m, 1H),
8.45 (dd, 1H, J = 6.5 Hz, 12.0 Hz)
$^{19}$F-NMR (solvent, deutero chloroform; external standard, $CF_3CO_2H$)
δ(ppm) 6.0 (m, 3F),
33.0 (m, 1F),
33.5 (s, 2F),
54.0 (m, 1F).

Production example 6

Using the same procedure as that described in Production example 1, 2.00 g of 2-fluoroacetanilide, 4.71 g of pentafluoropropionic acid and 4.86 g of xenon difluoride gave 0.45 g of 2-fluoro-4-pentafluoroethylacetanilide as white crystals.
Yield 12%
$^1$H-NMR (solvent, deutero chloroform; internal standard, TMS)
δ(ppm) 2.25 (s, 3H),
7.10-7.50 (m, 2H),
7.50-7.80 (m, 1H),
8.35-8.70 (m, 1H).
$^{19}$F-NMR (solvent, deutero chloroform; external standard, $CF_3CO_2H$)
δ(ppm) 6.0 (br., 3F),
35.1 (br., 2F),
49.5 (br., 1F).

Production example 7

Using the same procedure as that described in Production example 1, 2.00 g of 2,5-difluoroacetanilide, 5.83 g of heptafluorobutyric acid and 4.27 g of xenon difluoride gave 0.65 g of 2,5-difluoro-4-heptafluoropropylacetanilide as white crystals.
Yield 15%
$^1$H-NMR (solvent, deutero chloroform; internal standard, TMS)
δ(ppm) 2.25 (s, 3H),
7.05 (dd, 1H, J = 6.0 Hz, 12.0 Hz),
7.55-7.90 (br., 1H),
8.20 (dd, 1H, 3 = 6.5 Hz, 14.0 Hz).

Production example 8

Using the same procedure as that described in Production example 2, 0.30 g of 2,5-difluoro-4-pentafluoroethylacetanilide gave 0.25 g of 2,5-difluoro-4-pentafluoroethylaniline.
$^1$H-NMR (solvent, deutero chloroform; internal standard, TMS)
δ(ppm) 3.50-4.50 (br., 2H),
6.17 (dd, 1H, J = 6.0 Hz, 10.0 Hz),
6.55 (dd, 1H, J = 7.0 Hz, 12.0 Hz).

Production example 9

Using the same procedure as that described in Production example 2, 0.50 g of 2,5-difluoro-4-heptafluoropropylacetanilide gave 0.41 g of 2,5-difluoro-4-heptafluoropropylaniline.
$^1$H-NMR (solvent, deutero chloroform; internal standard, TMS)
δ(ppm) 4.20-4.80 (br., 2H),
6.60 (dd, 1H, J = 7.0 Hz, 11.0 Hz),
7.15 (dd, 1H, J = 6.0 Hz, 10.0 Hz).
$^{19}$F-NMR (solvent, deutero chloroform; external standard, $CF_3CO_2H$)
δ(ppm) 3.0 (t, 3F, J = 10.0 Hz),
30.0 (m, 2F),
38.1 (m, 1F),
47.4 (d, 2F, J = 20.0 Hz),
61.5 (m, 1F).

Production example 10

2.03 Grams of 4-trifluoromethylacetanilide was dissolved in 20 ml of dichloromethane, and to this solution was added 0.2 g of trifluoromethanesulfonic acid. Thereafter, 4.0 g of xenon difluoride was added in small portions with stirring and water-cooling. After addition, stirring was continued overnight at room temperature, and after adding water, the reaction mixture was extracted with 50 ml of dichloromethane. The dichloromethane layer was washed with water and dried over anhydrous sodium sulfate, and dichloromethane was removed by evaporation under reduced pressure to give 2.05 g of a product. This product was purified with column-chromatography on silica gel (solvent, chloroform : ethyl acetate = 20 : 1) to give 450 mg of 2-fluoro-4-trifluoromethylacetanilide.
Yield 20%
$^1$H-NMR (solvent, deutero chloroform; internal standard, TMS)
δ(ppm) 2.25 (s, 3H),
7.20-8.00 (m, 3H),
8.30-8.80 (m, 1H)
$^{19}$F-NMR (solvent, deutero chloroform; external standard, $CF_3CO_2H$)
δ(ppm) 15.0 (s, 3F),
-50.4 (m, 1F)

Formulation examples are described below. The present compounds are referred to by Compound No. in Table 1, and parts in the examples are by weight.

Formulation example 1

Ten parts of each of the present compounds (1) to (10), 14 parts of polyoxyethylene styrylphenyl ether, 6 parts of calcium dodecylbenzenesulfonate, 35 parts of xylene and 35 parts of dimethylformamide are well mixed to obtain an emulsifiable concentrate of each compound.

Formulation example 2

Twenty parts of each of the present compounds (1) to (10), 10 parts of fenitrothion, 3 parts of calcium lignosulfonate, 2 parts of sodium lauryl sulfate and 65 parts of synthetic hydrated silicon dioxide are well pulverized and mixed to obtain a wettable powder of each compound.

Formulation example 3

One part of each of the present compounds (1) to (10), 2 parts of carbaryl, 87 parts of kaolin clay and 10 parts of talc are well pulverized and mixed to obtain a dust of each compound.

Formulation example 4

Five parts of each of the present compounds (1) to (10), 1 part of synthetic hydrated silicon dioxide, 2 parts of calcium lignosulfonate, 30 parts of bentonite and 62 parts of kaolin clay are well pulverised and mixed. The resulting mixture is well kneaded with water, granulated and dried to obtain a granule of each compound.

Test examples are given below. The present compounds are referred to by Compound No. in Table 1,

and compounds used as a control are referred to by Compound symbol in Table 2.

Table 2

| Compound symbol | Structural formula | Name |
|---|---|---|
| (A) | 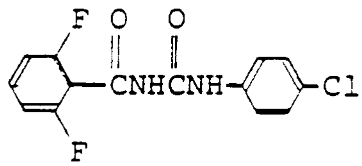 | Diflubenzuron(compound described in Japanese Patent Publication No. 18255/1977). |
| (B) | 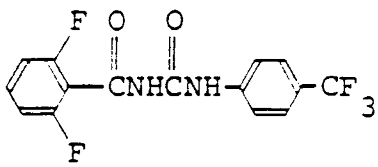 | Penfluron(compound described in Japanese Patent Publication No. 18255/1977). |
| (C) | 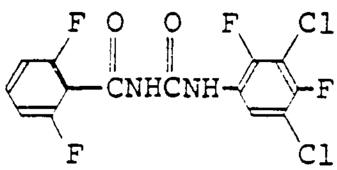 | Teflubenzuron [compound described in Japanese Patent Publication Kokai (Laid-open) No. 126460/1982]. |
| (D) | 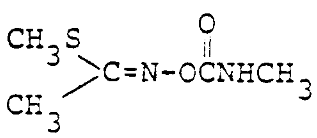 | Methomyl |
| (E) | 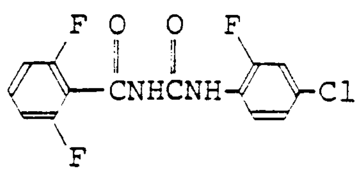 | Compound described in Japanese Patent Publication Kokai (Laid-open) No. 106454/1984. |
| (F) | 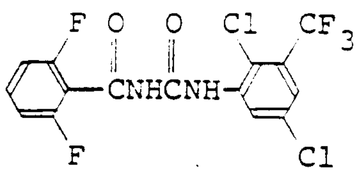 | Compound described in Japanese Patent Publication Kokai (Laid-open) No. 76452/1986. |

Test example 1

The emulsifiable concentrates of the following present compounds prepared according to Formulation example 1 were each diluted with water so that the active ingredient concentration was 3.5 ppm. Thereafter, 100 ml of the aqueous dilute solution thus obtained was poured into a 180-ml polyethylene cup, and 20 last instar larvae of the common mosquito (Culex pipiens pallens) were introduced into the cup. The larvae were bred on a bait until emergence to obtain an emergence inhibitory ratio (two replications).

The results are shown in Table 3.

Table 3

| Test compound | Emergence inhibitory ratio (%) |
|---|---|
| (1) | 100 |
| (2) | 100 |
| (3) | 100 |
| (4) | 100 |
| (5) | 100 |
| (6) | 100 |
| (7) | 100 |
| (8) | 100 |
| (9) | 100 |
| (10) | 100 |
| No treatment | 10 |

Test example 2

The emulsifiable concentrates of the following present compounds and controls prepared according to Formulation example 1 were each diluted with water to a prescribed concentration, and 2 ml of the aqueous dilute solution thus obtained was applied onto 13 g of artificial diet for tobacco cutworm (Spodoptera litura) which diet was then placed in a polyethylene cup of 11 cm in diameter. Ten fourth instar larvae of the tobacco cutworm were then introduced into the cup. After six days, the members of dead and alive were counted to obtain a % mortality value (two replications).

The results are shown in Table 4.

Table 4

| Test compound | Concentration and mortality (%) | |
|---|---|---|
| | 1.5 ppm | 0.5 ppm |
| (1) | 100 | 80 |
| (2) | 100 | 100 |
| (3) | 100 | 100 |
| (4) | 100 | 70 |
| (5) | 100 | 100 |
| (6) | 100 | 100 |
| (7) | 100 | 100 |
| (9) | 100 | 100 |
| (10) | 100 | 100 |
| (A) | 10 | 5 |
| (B) | 15 | 5 |
| (C) | 80 | 20 |
| (D) | 10 | 0 |
| (E) | 45 | 10 |
| (F) | 60 | 20 |
| No treatment | 5 | |

Test example 3

One ml each of the 67,000-fold aqueous dilute solutions (corresponding to 1.5 ppm) of emulsifiable concentrates prepared from the following present compounds and controls according to Formulation

example 1 was applied onto 5 g of artificial diet for rice stem borer (Chilo suppressalis) which had been previously prepared in a polyethylene cup of 5.5 cm in diameter. Ten 10-day old larvae of the rice stem borer were then introduced into the cup. After eight days, the number of dead and alive were counted to obtain a % mortality value (two replications).

The results are shown in Table 5.

Table 5

| Test compound | Mortality (%) |
|---|---|
| (1) | 90 |
| (2) | 100 |
| (3) | 90 |
| (4) | 100 |
| (5) | 100 |
| (6) | 80 |
| (7) | 100 |
| (8) | 80 |
| (9) | 90 |
| (10) | 80 |
| (A) | 30 |
| (B) | 40 |
| (C) | 45 |
| (D) | 5 |
| (E) | 35 |
| (F) | 10 |
| No treatment | 5 |

Test example 4

A circular cabbage leaf of 9.0 cm in diameter was dipped for 1 minute in the 200,000-fold aqueous dilute solutions each (corresponding to 0.5 ppm) of emulsifiable concentrates prepared from the following present compounds and controls according to Formulation example 1. After air-drying, the leaf was placed in a polyethylene cup of 11 cm in diameter in which a filter paper was placed on the bottom, and ten third instar larvae of the diamond-back moth (Plutella xylostella) of a field strain were introduced therein. After nine days, the number which had emerged was counted to obtain an emergence inhibitory ratio (two replications).

The results are shown in Table 6.

Table 6

| Test compound | Emergence inhibitory ratio (%) * |
|---|---|
| (1) | 100 |
| (2) | 100 |
| (3) | 89 |
| (4) | 89 |
| (5) | 100 |
| (6) | 100 |
| (7) | 100 |
| (8) | 73 |
| (9) | 100 |
| (10) | 100 |
| (A) | 11 |
| (B) | 33 |
| (C) | 33 |
| (D) | 0 |
| (E) | 33 |
| (F) | 22 |

- cont'd -

Table 6 (cont'd)

$$\text{* Emergence inhibitory ratio (\%)} = \left(1 - \frac{\text{Emergence inhibitory ratio in treated plot}}{\text{emergence inhibitory ratio in untreated plot}}\right) \times 100$$

## Claims

1. A benzoylurea derivative represented by the formula,

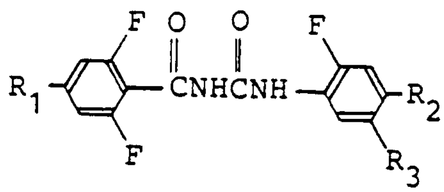

wherein $R_1$ is a hydrogen atom or a fluorine atom, $R_2$ is a trifluoromethyl group, a pentafluoroethyl group or a heptafluoropropyl group, and $R_3$ is a hydrogen atom, a fluorine atom or a chlorine atom.

2. The benzoylurea derivative according to Claim 1, wherein $R_1$ is a hydrogen atom or a fluorine atom, $R_2$ is a trifluoromethyl group, a pentafluoroethyl group or a heptafluoropropyl group, and $R_3$ is a hydrogen atom or a fluorine atom.

3. The benzoylurea derivative according to Claim 2 having the following formula,

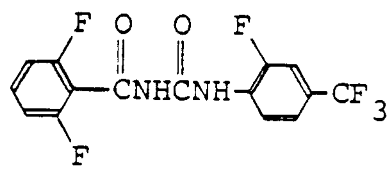

4. The benzoylurea derivative according to Claim 2 having the following formula,

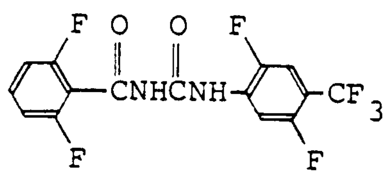

5. The benzoylurea derivative according to Claim 2 having the following formula,

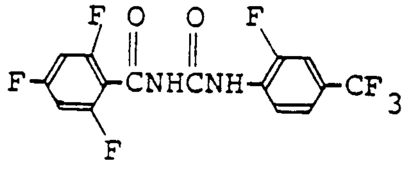

6. The benzoylurea derivative according to Claim 2 having the following formula,

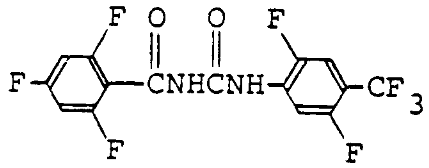

7. The benzoylurea derivative according to Claim 2 having the following formula,

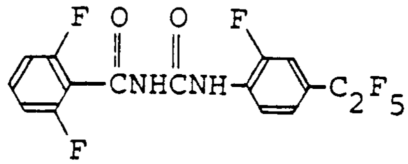

8. The benzoylurea derivative according to Claim 2 having the following formula,

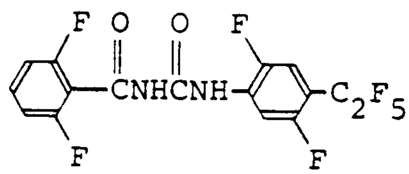

9. The benzoylurea derivative according to Claim 2 having the following formula,

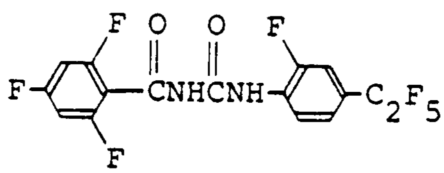

10. The benzoylurea derivative according to Claim 2 having the following formula,

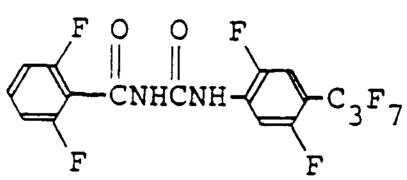

11. A method for producing a benzoylurea derivative represented by the formula,

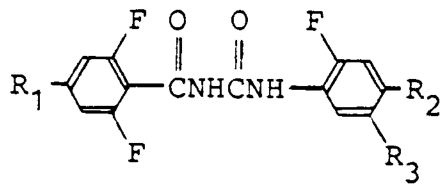

wherein $R_1$ is a hydrogen atom or a fluorine atom, $R_2$ is a trifluoromethyl group, a pentafluoroethyl group or a heptafluoropropyl group, and $R_3$ is a hydrogen atom, a fluorine atom or a chlorine atom, which comprises reacting a benzoylisocyanate compound represented by the formula,

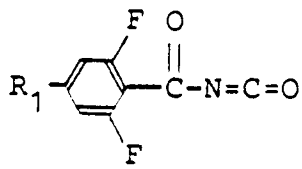

wherein $R_1$ is as defined above, with an aniline compound represented by the formula,

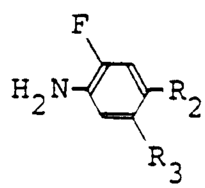

wherein $R_2$ and $R_3$ are as defined above.

**12.** A method for producing a benzoylurea derivative represented by the formula,

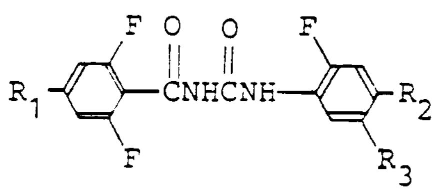

wherein $R_1$ is a hydrogen atom or a fluorine atom, $R_2$ is a trifluoromethyl group, a pentafluoroethyl group or a heptafluoropropyl group, and $R_3$ is a hydrogen atom, a fluorine atom or a chlorine atom, which comprises reacting a benzamide compound represented by the formula,

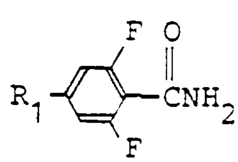

wherein $R_1$ is as defined above, with an isocyanate compound represented by the formula,

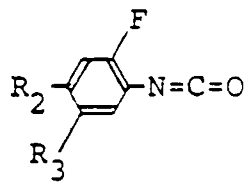

wherein $R_2$ and $R_3$ are as defined above.

**13.** An insecticidal composition which comprises as an active ingredient an insecticidally effective amount of a benzoylurea derivative represented by the formula,

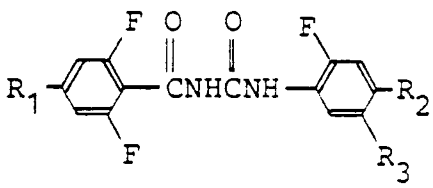

wherein $R_1$ is a hydrogen atom or a fluorine atom, $R_2$ is a trifluoromethyl group, a pentafluoroethyl group or a heptafluoropropyl group, and $R_3$ is a hydrogen atom, a fluorine atom or a chlorine atom, and an inert carrier or diluent.

**14.** The insecticidal composition according to Claim 13, wherein in the formula representing the benzoylurea derivative, $R_1$ is a hydrogen atom or a fluorine atom, $R_2$ is a trifluoromethyl group, a pentafluoroethyl group or a heptafluoropropyl group, and $R_3$ is a hydrogen atom or a fluorine atom.

**15.** A method for controlling insect pests which comprises applying an insecticidally effective amount of the benzoylurea derivative according to Claim 1.

**16.** Use of the benzoylurea derivative according to Claim 1 as an insecticide.

**17.** An aniline compound represented by the formula,

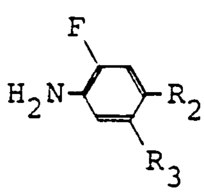

wherein $R_2$ is a trifluoromethyl group, a pentafluoroethyl group or a heptafluoropropyl group, and $R_3$ is a hydrogen atom, a fluorine atom or a chlorine atom, provided that when $R_2$ is a trifluoromethyl group, $R_3$ is a fluorine atom or a chlorine atom.

**18.** The aniline compound according to Claim 17 having the following formula,

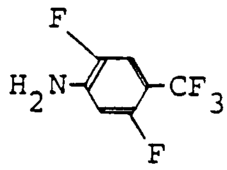

19. The aniline compound according to Claim 17 having the following formula,

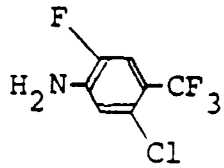

20. The aniline compound according to Claim 17 having the following formula,

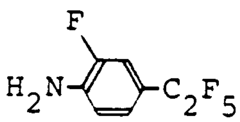

21. The aniline compound according to Claim 17 having the following formula,

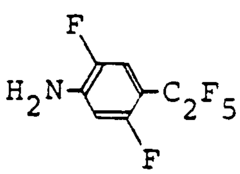

22. The aniline compound according to Claim 17 having the following formula,

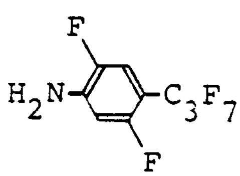

23. An isocyanate compound represented by the formula,

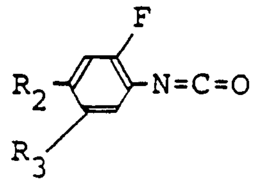

wherein $R_2$ is a trifluoromethyl group, a pentafluoroethyl group or a heptafluoropropyl group, and $R_3$ is a hydrogen atom, a fluorine atom or a chlorine atom.

**24.** The isocyanate compound according to Claim 23 having the following formula,

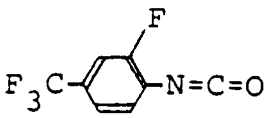

**25.** The isocyanate compound according to Claim 23 having the following formula,

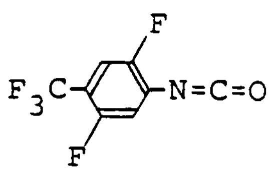

**26.** The isocyanate compound according to Claim 23 having the following formula,

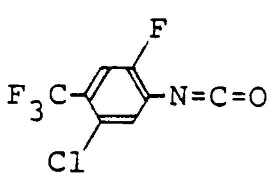

**27.** The isocyanate compound according to Claim 23 having the following formula,

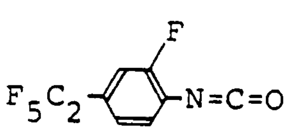

**28.** The isocyanate compound according to Claim 23 having the following formula,

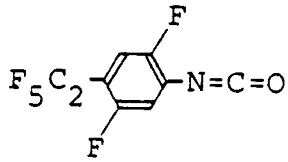

29. The isocyanate compound according to Claim 23 having the following formula,

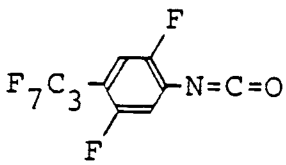

30. A method for producing an aniline compound represented by the formula,

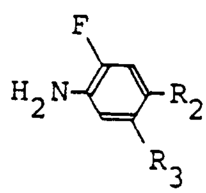

wherein $R_2$ is a trifluoromethyl group, a pentafluoroethyl group or a heptafluoropropyl group, and $R_3$ is a hydrogen atom, a fluorine atom or a chlorine atom, which comprises reacting an acetanilide compound represented by the formula,

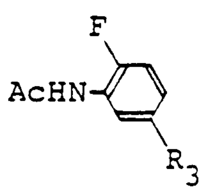

wherein $R_3$ is as defined above, with a carboxylic acid represented by the formula,

$R_2CO_2H$

wherein $R_2$ is as defined above, in the presence of xenon difluoride, to afford the resulting acetanilide compound represented by the formula,

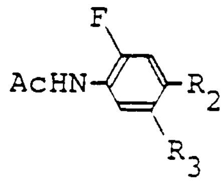

wherein $R_2$ and $R_3$ are as defined above, followed by deacetylation.

**31.** A method for producing an isocyanate compound represented by the formula,

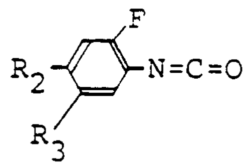

wherein $R_2$ is a trifluoromethyl group, a pentafluoroethyl group or a heptafluoropropyl group, and $R_3$ is a hydrogen atom, a fluorine atom or a chlorine atom, which comprises reacting an aniline compound represented by the formula,

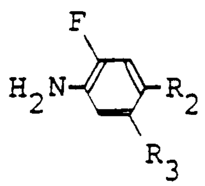

wherein $R_2$ and $R_3$ are as defined above, with phosgene.

## Revendications

**1.** Dérivé de benzoylurée, représenté par la formule

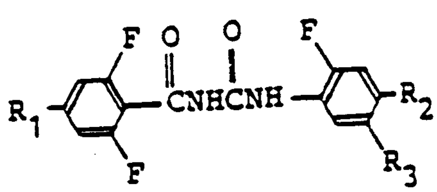

dans laquelle $R_1$ est un atome d'hydrogène ou un atome de fluor, $R_2$ est un groupement trifluorométhyle, un groupement pentafluoroéthyle ou un groupement heptafluoropropyle, et $R_3$ est un atome d'hydrogène, un atome de fluor ou un atome de chlore.

**2.** Dérivé de benzoylurée selon la revendication 1, dans lequel $R_1$ est un atome d'hydrogène ou un atome de fluor, $R_2$ est un groupement trifluorométhyle, un groupement pentafluoroéthyle ou un groupement heptafluoropropyle, et $R_3$ est un atome d'hydrogène ou un atome de fluor.

3. Dérivé de benzoylurée selon la revendication 2 répondant à la formule suivante

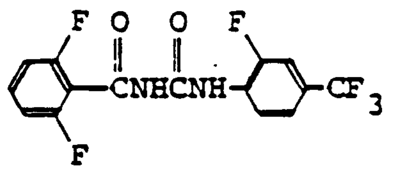

4. Dérivé de benzoylurée selon la revendication 2 répondant à la formule suivante

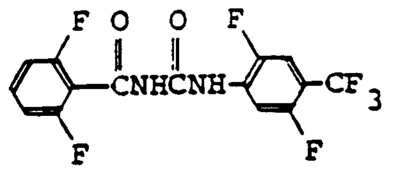

5. Dérivé de benzoylurée selon la revendication 2 répondant à la formule suivante

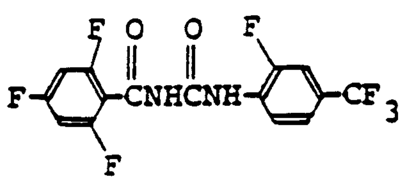

6. Dérivé de benzoylurée selon la revendication 2 répondant à la formule suivante

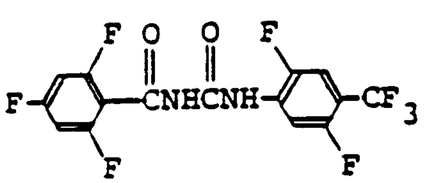

7. Dérivé de benzoylurée selon la revendication 2 répondant à la formule suivante

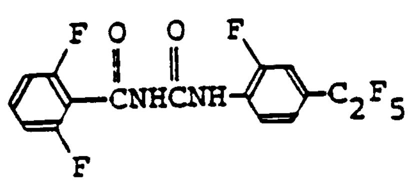

8. Dérivé de benzoylurée selon la revendication 2 répondant à la formule suivante

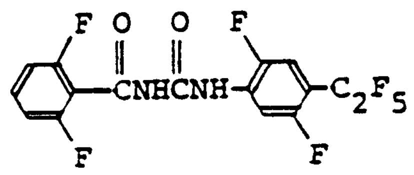

9. Dérivé de benzoylurée selon la revendication 2 répondant à la formule suivante

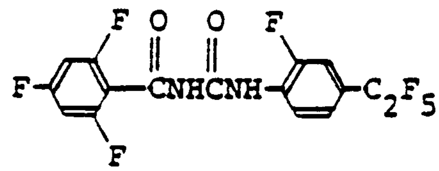

10. Dérivé de benzoylurée selon la revendication 2 répondant à la formule suivante

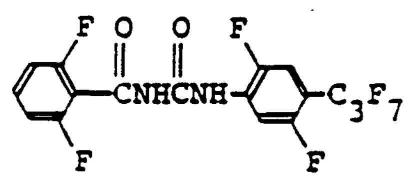

11. Procédé de préparation d'un dérivé de benzoylurée représenté par la formule

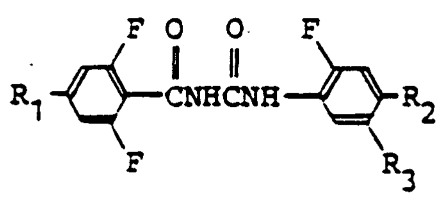

dans laquelle $R_1$ est un atome d'hydrogène ou un atome de fluor, $R_2$ est un groupement trifluoromethyle, un groupement pentafluoroéthyle ou un groupement heptafluoropropyle, et $R_3$ est un atome d'hydrogène, un atome de fluor ou un atome de chlore, comprenant la mise en réaction d'un dérivé d'isocyanate de benzoyle représenté par la formule

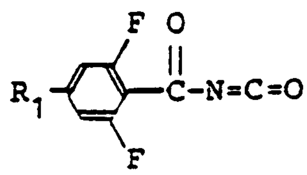

dans laquelle $R_1$ est tel que défini précédemment, avec un dérivé d'aniline représenté par la formule

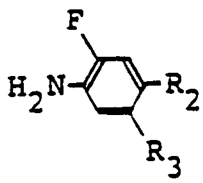

dans laquelle $R_2$ et $R_3$ sont tels que définis précédemment.

**12.** Procédé de préparation d'un dérivé de benzoylurée représenté par la formule

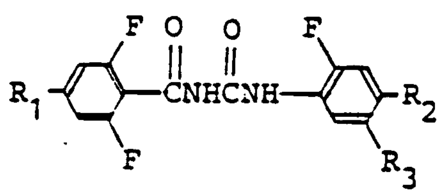

dans laquelle $R_1$ est un atome d'hydrogène ou un atome de fluor, $R_2$ est un groupement trifluoromethyle, un groupement pentafluoroéthyle ou un groupement heptafluoropropyle, et $R_3$ est un atome d'hydrogène, un atome de fluor ou un atome de chlore, comprenant la mise en réaction d'un dérivé de benzamide représenté par la formule

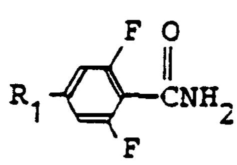

dans laquelle $R_1$ est tel que défini précédemment, avec un dérivé d'isocyanate représenté par la formule

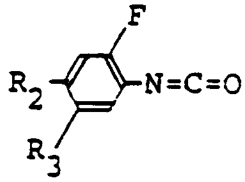

dans laquelle $R_2$ et $R_3$ sont tels que définis précédemment.

**13.** Composition insecticide qui comprend, en tant qu'ingrédient actif, une quantité efficace en tant qu'insecticide d'un dérivé de benzoylurée représenté par la formule

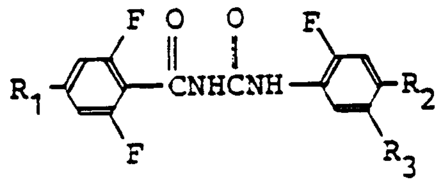

dans laquelle $R_1$ est un atome d'hydrogène ou un atome de fluor, $R_2$ est un groupement trifluorométhyle, un groupement pentafluoroéthyle ou un groupement heptafluoropropyle, et $R_3$ est un atome d'hydrogène, un atome de fluor ou un atome de chlore, ainsi qu'un véhicule ou diluant inerte.

**14.** Composition insecticide selon la revendication 13, dans laquelle, dans la formule représentant le dérivé de benzoylurée, $R_1$ est un atome d'hydrogène ou un atome de fluor, $R_2$ est un groupement trifluorométhyle, un groupement pentafluoroéthyle ou un groupement heptafluoropropyle, et $R_3$ est un atome d'hydrogène ou un atome de fluor.

**15.** Procédé de lutte contre les insectes nuisibles, consistant à appliquer une quantité efficace en tant qu'insecticide du dérivé de benzoylurée selon la revendication 1.

**16.** Utilisation du dérivé de benzoylurée selon la revendication 1 comme insecticide.

**17.** Dérivé d'aniline représenté par la formule

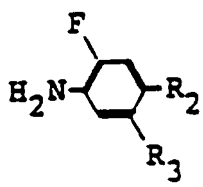

dans laquelle $R_2$ est un groupement trifluorométhyle, un groupement pentafluoroéthyle ou un groupement heptafluoropropyle, et $R_3$ est un atome d'hydrogène, un atome de fluor ou un atome de chlore, étant spécifié que quand $R_2$ est un groupement trifluorométhyle, $R_3$ est un atome de fluor ou un atome de chlore.

**18.** Dérivé d'aniline selon la revendication 17, répondant à la formule suivante

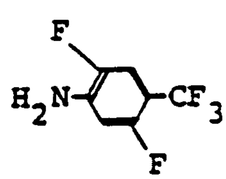

**19.** Dérivé d'aniline selon la revendication 17, répondant à la formule suivante

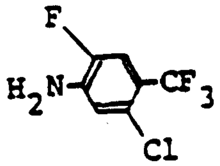

**20.** Dérivé d'aniline selon la revendication 17, répondant à la formule suivante

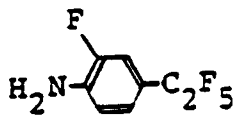

**21.** Dérivé d'aniline selon la revendication 17, répondant à la formule suivante

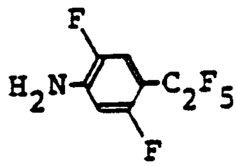

**22.** Dérivé d'aniline selon la revendication 17, répondant à la formule suivante

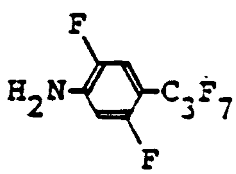

**23.** Dérivé d'isocyanate représenté par la formule

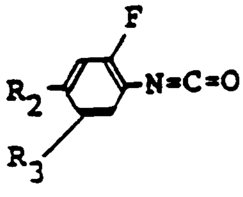

dans laquelle $R_2$ est un groupement trifluorométhyle, un groupement pentafluoroéthyle ou un groupement heptafluoropropyle, et $R_3$ est un atome d'hydrogène, un atome de fluor ou un atome de chlore.

**24.** Dérivé d'isocyanate selon la revendication 23, répondant à la formule suivante

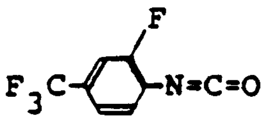

**25.** Dérivé d'isocyanate selon la revendication 23, répondant à la formule suivante

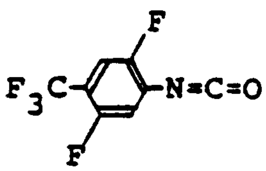

**26.** Dérivé d'isocyanate selon la revendication 23, répondant à la formule suivante

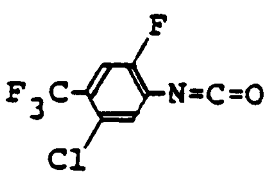

**27.** Dérivé d'isocyanate selon la revendication 23, répondant à la formule suivante

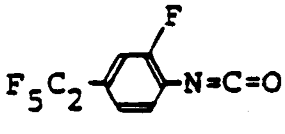

**28.** Dérivé d'isocyanate selon la revendication 23, répondant à la formule suivante

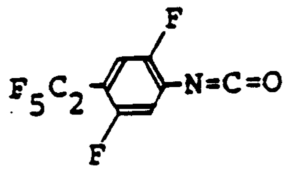

**29.** Dérivé d'isocyanate selon la revendication 23, répondant à la formule suivante

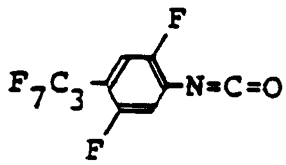

**30.** Procédé de préparation d'un dérivé d'aniline représenté par la formule

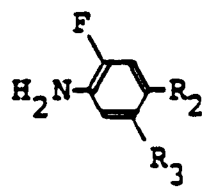

dans laquelle $R_2$ est un groupement trifluorométhyle, un groupement pentafluoroéthyle ou un groupement heptafluoropropyle, et $R_3$ est un atome d'hydrogène, un atome de fluor ou un atome de chlore, comprenant la mise en réaction d'un dérivé d'acétanilide représenté par la formule

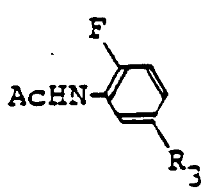

dans laquelle $R_3$ est tel que défini précédemment, avec un acide carboxylique représenté par la formule

$R_2CO_2H$

dans laquelle $R_2$ est tel que défini précédemment, en présence de difluorure de xénon, pour obtenir le dérivé d'acétanilide résultant, représenté par la formule

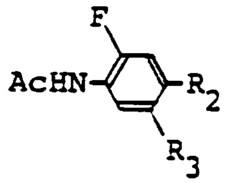

dans laquelle $R_2$ et $R_3$ sont tels que définis précédemment, suivie de désacétylation.

**31.** Procédé de préparation d'un dérivé d'isocyanate représenté par la formule

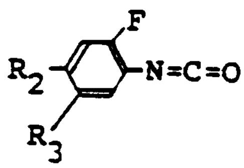

dans laquelle $R_2$ est un groupement trifluorométhyle, un groupement pentafluoroéthyle ou un groupement heptafluoropropyle, et $R_3$ est un atome d'hydrogène, un atome de fluor ou un atome de chlore, comprenant la mise en réaction d'un dérivé d'aniline représenté par la formule

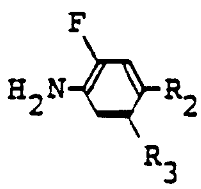

dans laquelle $R_2$ et $R_3$ sont tels que définis précédemment, avec du phosgène.

**Patentansprüche**

**1.** Benzoylharnstoffderivat der Formel

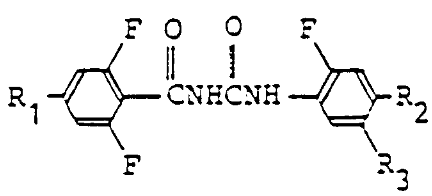

in der $R_1$ ein Wasserstoff- oder Fluoratom bedeutet, $R_2$ eine Trifluormethyl-, Pentafluorethyl- oder Heptafluorpropylgruppe bedeutet und $R_3$ ein Wasserstoff-, Fluor- oder Chloratom darstellt.

**2.** Benzoylharnstoffderivat nach Anspruch 1, wobei $R_1$ ein Wasserstoff- oder Fluoratom bedeutet, $R_2$ eine Trifluormethyl-, Pentafluorethyl- oder Heptafluorpropylgruppe darstellt und $R_3$ ein Wasserstoff- oder Fluoratom bedeutet.

**3.** Benzoylharnstoffderivat nach Anspruch 2 mit der nachstehenden Formel

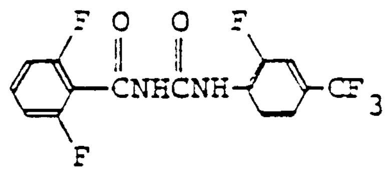

**4.** Benzoylharnstoffderivat nach Anspruch 2 mit der nachstehenden Formel

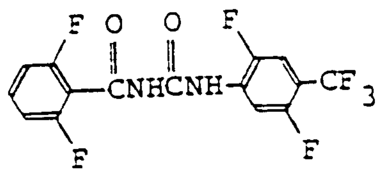

**5.** Benzoylharnstoffderivat nach Anspruch 2 mit der nachstehenden Formel

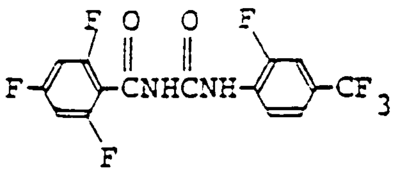

**6.** Benzoylharnstoffderivat nach Anspruch 2 mit der nachstehenden Formel

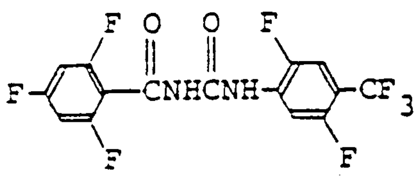

**7.** Benzoylharnstoffderivat nach Anspruch 2 mit der nachstehenden Formel

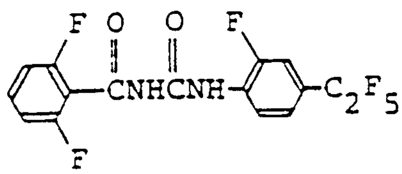

**8.** Benzoylharnstoffderivat nach Anspruch 2 mit der nachstehenden Formel

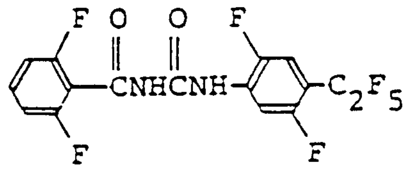

9. Benzoylharnstoffderivat nach Anspruch 2 mit der nachstehenden Formel

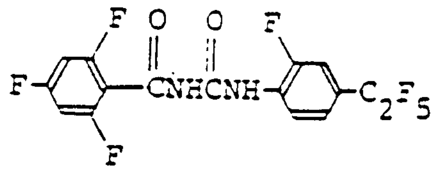

10. Benzoylharnstoffderivat nach Anspruch 2 mit der nachstehenden Formel

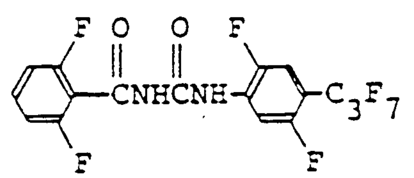

11. Verfahren zur Herstellung eines Benzoylharnstoffderivats der Formel

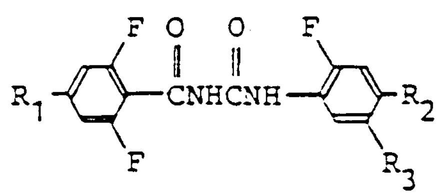

in der $R_1$ ein Wasserstoff- oder Fluoratom bedeutet, $R_2$ eine Trifluormethyl-, Pentafluorethyl- oder Heptafluorpropylgruppe bedeutet und $R_3$ ein Wasserstoff-, Fluor- oder Chloratom darstellt, umfassend die Umsetzung einer Benzoylisocyanatverbindung der Formel

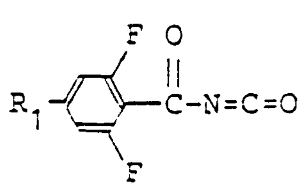

in der $R_1$ wie vorstehend definiert ist, mit einer Anilinverbindung der Formel

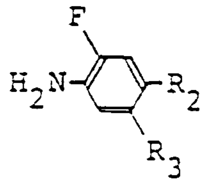

in der $R_2$ und $R_3$ wie vorstehend definiert sind.

**12.** Verfahren zur Herstellung eines Benzoylharnstoffderivats der Formel

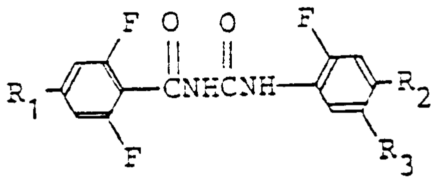

in der $R_1$ ein Wasserstoff- oder Fluoratom bedeutet, $R_2$ eine Trifluormethyl-, Pentafluorethyl- oder Heptafluorpropylgruppe bedeutet und $R_3$ ein Wasserstoff-, Fluor- oder Chloratom darstellt, umfassend die Umsetzung einer Benzamidverbindung der Formel

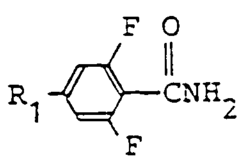

in der $R_1$ wie vorstehend definiert ist, mit einer Isocyanatverbindung der Formel

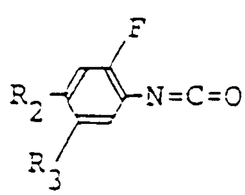

in der $R_2$ und $R_3$ wie vorstehend definiert sind.

**13.** Insektizid, umfassend als Wirkstoff eine insektizid wirksame Menge eines Benzoylharnstoffderivats der Formel

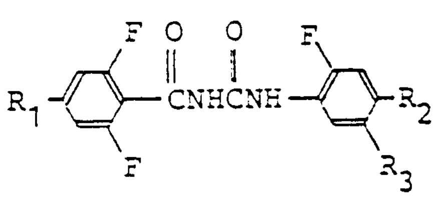

in der $R_1$ ein Wasserstoff- oder Fluoratom bedeutet, $R_2$ eine Trifluormethyl-, Pentafluorethyl- oder Heptafluorpropylgruppe bedeutet und $R_3$ ein Wasserstoff-, Fluor- oder Chloratom darstellt, und einen inerten Träger oder ein inertes Verdünnungsmittel.

**14.** Insektizid nach Anspruch 13, wobei in der Formel des Benzoylharnstoffderivats $R_1$ ein Wasserstoff- oder Fluoratom bedeutet, $R_2$ eine Trifluormethyl-, Pentafluorethyl- oder Heptafluorpropylgruppe darstellt und $R_3$ ein Wasserstoff- oder Fluoratom bedeutet.

**15.** Verfahren zur Bekämpfung von Schadinsekten, umfassend die Anwendung einer insektizid wirksamen Menge eines Benzoylharnstoffderivats nach Anspruch 1.

**16.** Verwendung des Benzoylharnstoffderivats nach Anspruch 1 als Insektizid.

**17.** Anilinverbindung der Formel

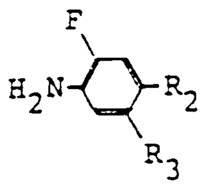

in der $R_2$ eine Trifluormethyl-, Pentafluorethyl- oder Heptafluorpropylgruppe bedeutet, und $R_3$ ein Wasserstoff-, Fluor- oder Chloratom darstellt, mit der Maßgabe, daß, falls $R_2$ eine Trifluormethylgruppe bedeutet, $R_3$ ein Fluor- oder Chloratom ist.

**18.** Anilinverbindung nach Anspruch 17 mit der nachstehenden Formel

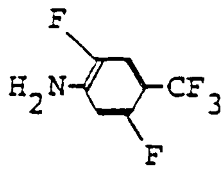

**19.** Anilinverbindung nach Anspruch 17 mit der nachstehenden Formel

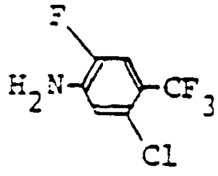

**20.** Anilinverbindung nach Anspruch 17 mit der nachstehenden Formel

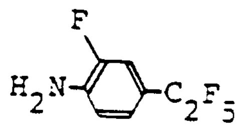

21. Anilinverbindung nach Anspruch 17 mit der nachstehenden Formel

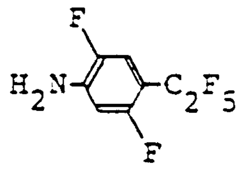

22. Anilinverbindung nach Anspruch 17 mit der nachstehenden Formel

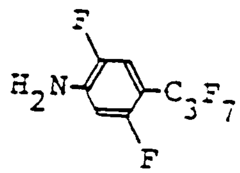

23. Isocyanatverbindung der Formel

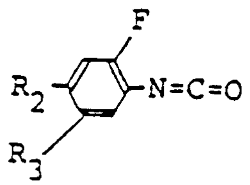

in der $R_2$ eine Trifluormethyl-, Pentafluorethyl- oder Heptafluorpropylgruppe bedeutet und $R_3$ ein Wasserstoff-, Fluor- oder Chloratom darstellt.

24. Isocyanatverbindung nach Anspruch 23 mit der nachstehenden Formel

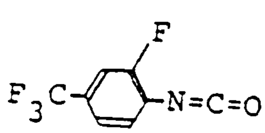

25. Isocyanatverbindung nach Anspruch 23 mit der nachstehenden Formel

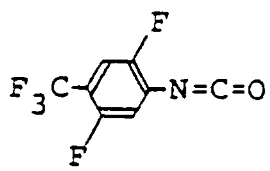

26. Isocyanatverbindung nach Anspruch 23 mit der nachstehenden Formel

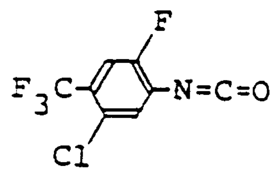

27. Isocyanatverbindung nach Anspruch 23 mit der nachstehenden Formel

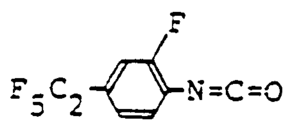

28. Isocyanatverbindung nach Anspruch 23 mit der nachstehenden Formel

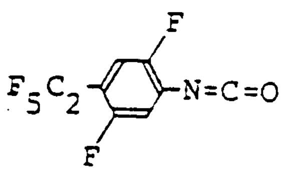

29. Isocyanatverbindung nach Anspruch 23 mit der nachstehenden Formel

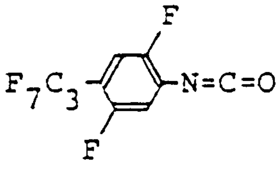

30. Verfahren zur Herstellung einer Anilinverbindung der Formel

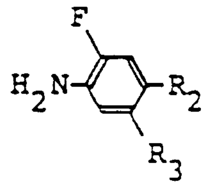

in der $R_2$ eine Trifluormethyl-, Pentafluorethyl- oder Heptafluorpropylgruppe bedeutet und $R_3$ ein Wasserstoff-, Fluor- oder Chloratom darstellt, umfassend die Umsetzung einer Acetanilidverbindung der Formel

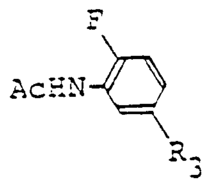

in der $R_3$ wie vorstehend definiert ist, mit einer Carbonsäure der Formel

$R_2CO_2H$

in der $R_2$ wie vorstehend definiert ist, in Gegenwart von Xenondifluorid, um als Ergebnis die Acetanilidverbindung der Formel

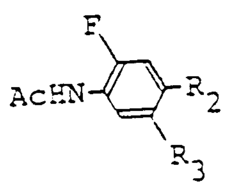

in der $R_2$ und $R_3$ wie vorstehend definiert sind, zu erhalten, gefolgt von einer Deacetylierung.

**31.** Verfahren zur Herstellung einer Isocyanatverbindung der Formel

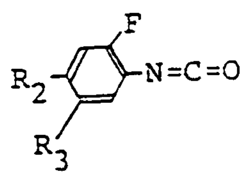

in der $R_2$ eine Trifluormethyl-, Pentafluorethyl- oder Heptafluorpropylgruppe bedeutet und $R_3$ ein Wasserstoff-, Fluor- oder Chloratom darstellt, umfassend die Umsetzung einer Anilinverbindung der Formel

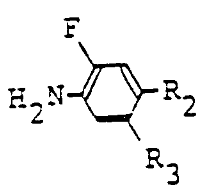

in der $R_2$ und $R_3$ wie vorstehend definiert sind, mit Phosgen.